(19) **Europäisches Patentamt**

**European Patent Office**

**Office européen des brevets**

(11) **EP 0 907 752 B1**

(12) **EUROPEAN PATENT SPECIFICATION**

(45) Date of publication and mention
of the grant of the patent:
**26.06.2002 Bulletin 2002/26**

(51) Int Cl.⁷: **C12Q 1/68**

(21) Application number: **97922519.0**

(22) Date of filing: **29.04.1997**

(86) International application number:
**PCT/US97/07134**

(87) International publication number:
**WO 97/41258 (06.11.1997 Gazette 1997/47)**

(54) **METHOD FOR DETERMINATION OF NUCLEIC ACID SEQUENCES AND DIAGNOSTIC APPLICATIONS THEREOF**

VERFAHREN ZUR BESTIMMUNG VON NUKLEINSÄURE-SEQUENZEN UND DIAGNOSTISCHE ANWENDUNGEN DERSELBEN

PROCEDE DE DETERMINATION DE SEQUENCES D'ACIDES NUCLEIQUES ET LEURS APPLICATIONS DIAGNOSTIQUES

(84) Designated Contracting States:
**DE FR GB**

(30) Priority: **01.05.1996 US 640672**
**19.07.1996 US 684498**

(43) Date of publication of application:
**14.04.1999 Bulletin 1999/15**

(73) Proprietor: **VISIBLE GENETICS INC.**
**Toronto, Ontario M5G 1Z6 (CA)**

(72) Inventors:
 • **LEUSHNER, James**
 **North York, Ontario M5M 4M3 (CA)**
 • **HUI, May**
 **Toronto, Ontario M4Y 1G1 (CA)**
 • **DUNN, James, M.**
 **Scarborough, Ontario M1K 2S8 (CA)**
 • **LARSON, Marina T.**
 **Silverthorne, Colorado 80498 (US)**
 • **LACROIX, Jean-Michel**
 **Etobicoke, Ontario M8Z 5A3 (CA)**

(74) Representative:
**Wibbelmann, Jobst, Dr., Dipl.-Chem.**
**Wuesthoff & Wuesthoff,**
**Patent- und Rechtsanwälte,**
**Schweigerstrasse 2**
**81541 München (DE)**

(56) References cited:
 **WO-A-93/08305**          **WO-A-96/01908**
 **WO-A-96/01909**          **US-A- 5 405 746**

 • **ANALYTICAL BIOCHEMISTRY, vol. 216, no. 1, 1 January 1994, pages 1-14, XP000420647 VENIGALLA B RAO: "DIRECT SEQUENCING OF POLYMERASE CHAIN REACTION-AMPLIFIED DNA"**
 • **ANALYTICAL BIOCHEMISTRY, vol. 224, no. 1, 1 January 1995, pages 117-121, XP000486746 WIEMANN S ET AL: "SIMULTANEOUS ON-LINE DNA SEQUENCING ON BOTH STRANDS WITH TWO FLUORESCENT DYES"**
 • **NATURE, vol. 376, 31 August 1995, page 796/797 XP000606193 REEVE M A ET AL: "A NOVEL THERMOSTABLE POLYMERASE FOR DNA SEQUENCING" cited in the application**
 • **PCR METHODS & APPLICATIONS, vol. 3, no. 5, April 1994, pages S107-S112, XP000606764 KRETZ K ET AL: "CYCLE SEQUENCING"**

**Description**

BACKGROUND OF THE INVENTION

This application relates to DNA sequencing reactions, and in particular to improved sequencing reaction protocols making use of thermally stable polymerase enzymes having enhanced capacity to incorporate chain terminating nucleotides during chain termination sequencing reactions.

DNA sequencing is generally performed using techniques based on the "chain termination" method described by Sanger et al., *Proc. Nat'l Acad. Sci. (USA)* 74(12): 5463-5467 (1977). Basically, in this process, DNA to be tested is isolated, rendered single stranded, and placed into four vessels. Each vessel contains the necessary components to replicate the DNA strand, i.e., a template-dependant DNA polymerase, a short primer molecule complementary to a known region of the DNA to be sequenced, and individual nucleotide triphosphates in a buffer conducive to hybridization between the primer and the DNA to be sequenced and chain extension of the hybridized primer. In addition, each vessel contains a small quantity of one type of dideoxynucleotide triphosphate, e.g. dideoxyadenosine triphosphate (ddA).

In each vessel, each piece of the isolated DNA is hybridized with a primer. The primers are then extended, one base at a time to form a new nucleic acid polymer complementary to the isolated pieces of DNA. When a dideoxynucleotide is incorporated into the extending polymer, this terminates the polymer strand and prevents it from being further extended. Accordingly, in each vessel, a set of extended polymers of specific lengths are formed which are indicative of the positions of the nucleotide corresponding to the dideoxynucleic acid in that vessel. These sets of polymers are then evaluated using gel electrophoresis to determine the sequence.

Improvements to the original technique described by Sanger et al. have included improvements to the enzyme used to extend the primer chain. For example, Tabor et al. have described enzymes such as T7 DNA polymerase which have increased processivity, and increased levels of incorporation of dideoxynucleotides. *(See* US Patent No. 4,795,699 and EP-A1-0 655 506. More recently, Reeve et al. have described a thermostable enzyme preparation, called Thermo Sequenase™, with many of the properties of T7 DNA polymerase. *Nature* 376: 796-797 (1995). The literature supplied with the Thermo Sequenase™ product suggests dividing a DNA sample containing 0.5-2 μg of single stranded DNA (or 0.5 to 5 μg of double stranded DNA) into four aliquots, and combining each aliquot with the Thermo Sequenase™ enzyme preparation, one dideoxynucleotide termination mixture containing one ddNTP and all four dNTP's; and a dye-labeled primer which will hybridize to the DNA to be sequenced. The mixture is placed in a thermocycler and run for 20-30 cycles of annealing, extension and denaturation to produce measurable amounts of dye-labeled extension products of varying lengths which are then evaluated by gel electrophoresis.

In addition, the processes known for determining the sequence of DNA can be preceded by amplification of a selected portion of the genetic material in a sample to enrich the concentration of a region of interest relative to other DNA. For example, it is possible to amplify a selected portion of a gene using a polymerase chain reaction (PCR) as described in U.S. Patents Nos. 4,683,194, 4,683,195 and 4,683,202. This process involves the use of pairs of primers, one for each strand of the duplex DNA, that will hybridize at a site located near a region of interest in a gene. Chain extension polymerization (without a chain terminating nucleotide) is then carried out in repetitive cycles to increase the number of copies of the region of interest many times. The amplified polynucleotides are then separated from the reaction mixture and used as the starting sample for the sequencing reaction. Gelfand et al. have described a thermostable enzyme, "Taq polymerase," derived from the organism *Thermus aquaticus*, which is useful in this amplification process. (*See* US Patent Nos. 5,352,600 and 5,079.352.)

U.S. Patent No. 5,427,911, describes a process for coupled amplification and sequencing of DNA. In this process, a sample is combined with two primers and amplified for a number of cycles to achieve 10,000 to 100,000-fold amplification of a selected region of the initial genomic DNA. Thereafter, the sample is divided into 8 test and 2 control aliquots. The test aliquots each receive one type of dideoxynucleotide triphosphates and a labeled primer complementary to one of the amplified DNA strands. Thus, the eight test aliquots taken together provide one reaction for each base type in each sequencing direction.

Notwithstanding efforts to develop the state of the sequencing art, the cost and complexity of the sequencing process has been generally viewed as making it unavailable for use in routine diagnostics where the sequence of the same region of DNA is determined over and over again in multiple patients. International Patent Publications Nos. WO 96/01908, WO 96/01909, and WO 96/07761, disclose a hierarchical approach which permits more cost effective utilization of sequencing techniques in diagnostics by combining sequencing with less expensive initial procedures, and only performing sequencing to the extent it is necessary. For example, for diagnosis of mutations in the retinoblastoma gene, WO 96/01908 discloses a protocol in which multiplex amplification is first performed and used to detect certain types of mutations. Only if the multiplex amplification procedure fails to identify a mutation is sequencing performed. However, the procedure described for sequencing requires a separate amplification of the exon to be sequenced, followed by the sequencing reaction and analysis of the sequencing fragments.

It would be desirable to have a method for the diagnostic sequencing of genetic materials which could be performed with fewer steps than the methods known in the art. This reduction of steps would reduce the cost and complexity of the assay, as well as reducing the number of opportunities for sample contamination or handling error. Such a method might suitably be performed in a single vessel, therefore making it more suited to automation. It is the object of the present invention to provide such a method.

SUMMARY OF THE INVENTION

The present invention is based on the observation and discovery that the addition of a reaction mixture containing the thermostable polymerase Thermo Sequenase™, two primers which bind to complementary strands of a target DNA molecule at sites flanking a region of interest in the target DNA molecule, a mixture of nucleotide triphosphates (A, C, G and T) and a dideoxynucleotide triphosphate to a DNA sample containing even relatively small amounts of target DNA in combination with substantial amounts of non-target DNA and the processing of the combination through multiple cycles of annealing, extension and denaturation results in the production of a mixture which can be loaded directly onto a gel for sequence analysis. This result makes it possible to perform sequencing reactions directly on complex DNA mixtures, such as the products of multiplex amplification reactions, which could not previously be used for sequencing without intermediate amplification and/or purification to increase the amount of target DNA present relative to other DNA species. Thus, one aspect of the present invention is a method for determining the positions of at least one base within a selected region of a target nucleic acid polymer in a complex mixture of DNA comprising the steps of

(a) combining the mixture with a reaction mixture comprising all four types of deoxynucleotide triphosphates, one type of dideoxynucleotide triphosphate corresponding to the base to be determined, first and second primers and a thermally stable polymerase enzyme which incorporates dideoxynucleotides into an extending nucleic acid polymer at a rate which is no less than about 0.4 times the rate of incorporation of deoxynucleotides in an amplification mixture for a plurality of amplification cycles to form a reaction mixture, said first and second primers binding to the sense and antisense strands, respectively, of the target nucleic acid polymer for amplification of the selected region;
(b) exposing the reaction mixture to a plurality of temperature cycles each of which includes at least a high temperature denaturation phase and a lower temperature extension phase to produce a product mixture comprising sequencing fragments which are terminated by incorporation of the dideoxynucleotide; and
(c) evaluating product mixture to determine the lengths of the sequencing fragments produced.

This method can be used as part of a diagnostic method for analyzing a plurality of regions within a nucleic acid-containing sample for the presence of mutations by first performing a multiplex amplification reaction on the nucleic acid-containing sample using a plurality of primer pairs, one pair for each of the plurality of regions to be analyzed to produce a mixture of amplified fragments. The fragments are analyzed in accordance with the experimental protocol being employed, for example by a fragment length analysis. Thereafter, sequencing determinations are made by combining at least one aliquot of the mixture of amplified fragments with a sequencing mixture comprising first and second sequencing primers that bind to the sense and antisense strands, respectively, of one of the amplified regions, a nucleotide triphosphate feedstock mixture, a chain-terminating nucleotide triphosphate, and a thermally stable polymerase enzyme which incorporates dideoxynucleotides into an extending nucleic acid polymer at a rate which is no less than 0.4 times the rate of incorporation of deoxynucleotides to form a sequencing reaction mixture. This sequencing reaction mixture is then exposed to a plurality of temperature cycles each of which includes at least a high temperature denaturation phase and a lower temperature extension phase, thereby producing a plurality of terminated fragments which are evaluated to determine the positions of the chain-terminating nucleotide within the extending polymer.

BRIEF DESCRIPTION OF THE DRAWINGS

Figs. 1A and 1B illustrates a diagnostic method which incorporates the amplification and sequencing process of the present invention with a fragment-based analysis;
Fig. 2 illustrates the method of the invention schematically;
Figs. 3A - B show a comparison of sequencing runs performed using Thermo Sequenase™ as the polymerase in the method of the invention with results obtained using other thermostable polymerases in comparative experiments; and
Fig. 4 shows the data trace of Fig. 3A in greater detail.

DETAILED DESCRIPTION OF THE INVENTION

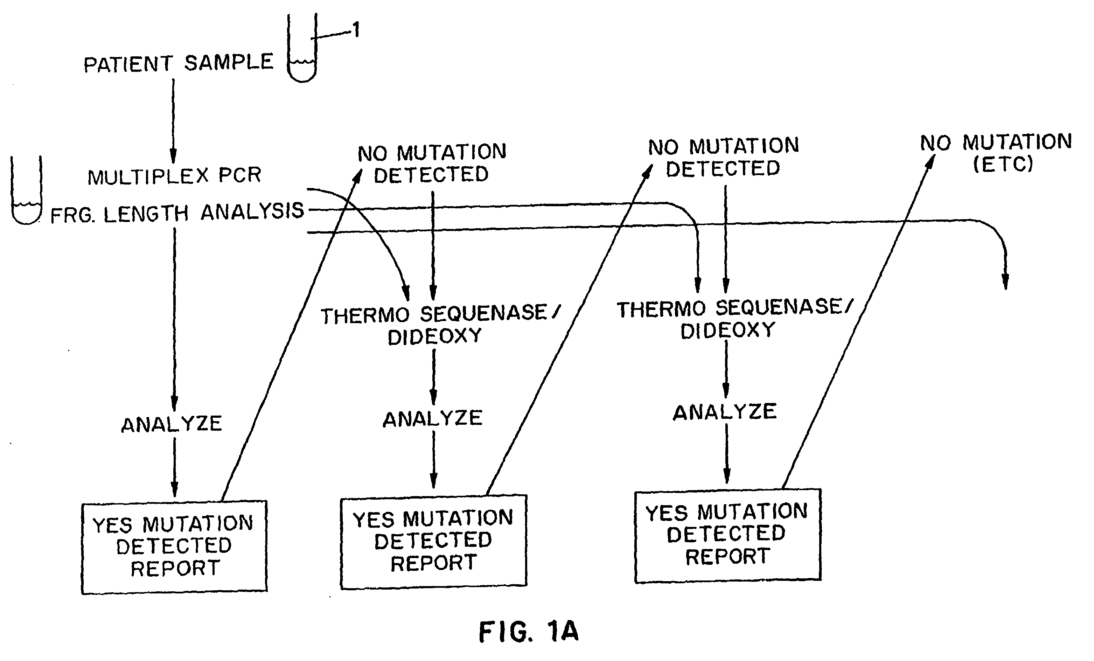

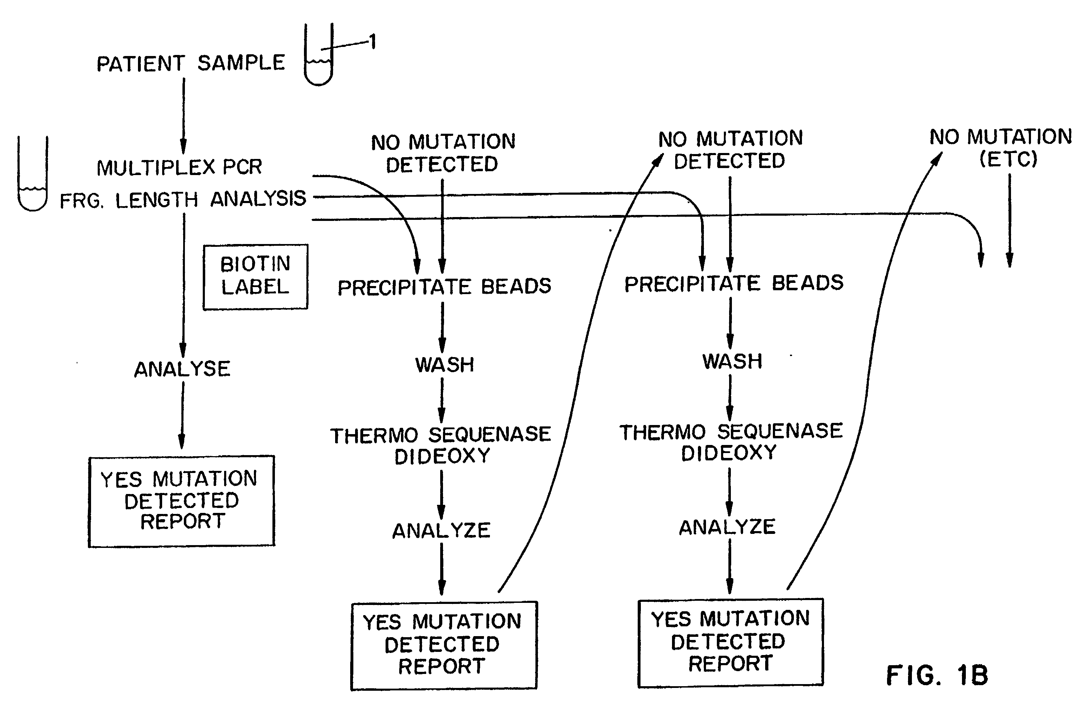

Figs. 1A and 1B illustrate the method of the present invention as part of an overall diagnostic strategy in which a series of analytical techniques may be performed on the same sample in a hierarchical analytical structure that depends on the outcome of the initial test. As shown, a patient sample 1 is first subjected to multiplex PCR to produce a complex mixture of amplification products. Since many clinically significant conditions, for example mutations in the RB1 gene, may involve deletions which are readily and inexpensively detectable by performing fragment length analysis on this mixture, a first step in many hierarchical analysis will be such a fragment length analysis.

According to the hierarchical model, if the results of the fragment length analysis fail to show a mutation, the sample 1 is further analyzed, for example by sequencing a selected exon. Prior to the present invention, this required the individual amplification of the selected exon from the sample because the there was not enough template in the initial multiplex PCR reaction to serve as a sequencing template. Using the method of the present invention, however, an aliquot of the original multiplex PCR amplification mixture can be used as the starting material for the sequencing method of the invention. Thus, the multiplex PCR amplification mixture is combined with two primers (at least one of which is labeled) for the region to be sequenced, a reaction mixture containing a polymerase such as Thermo Sequenase™ with a high affinity for incorporating dideoxynucleotide triphosphates into the extending polymer, and deoxy and dideoxynucleotide triphosphates and then processed for multiple cycles to produce the sequencing fragment mixture for analysis. In one embodiment, the multiplex amplification PCR is performed using capturable primers (for example biotin-labeled primers) and separated from the multiplex amplification reagents using affinity beads (e.g. avidin-coated beads) prior to the addition of the amplification/ sequencing reagents. (Fig. 1B). Additional aliquots of the multiplex reaction mixture may be processed to sequence different regions if no mutation is detected in the first sequencing step.

The method of the invention utilizes the properties of enzymes like Thermo Sequenase™, namely the ability to incorporate dideoxynucleotides into an extending polynucleotide at a rate which is no less than about 0.4 times the rate of incorporation of deoxynucleotides, to provide a method for the sequencing of a nucleic acid polymer from a multiplex amplification reaction product in a single vessel with a single set of thermocycling reactions and a single set of reagents. Thus, the method of the invention is ideally suited for automation.

The invention of the present application makes use of these properties in the context of analysis of a multiplex amplification mixture, however the general method on which this application is based permits the analysis of a wide range of complex mixtures of DNA which were previously inaccessible to single vessel/single reagent set sequencing procedures. These applications, which are addressed in concurrently filed applications, include the analysis of sequences directly from highly complex DNA such as genomic human DNA, without a preliminary selective amplification or purification step, and the detection and typing of microorganisms in a sample.

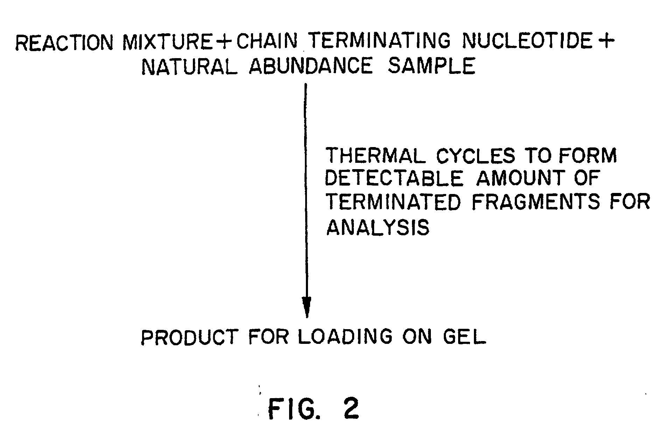

Fig. 2 illustrates the fundamental simplicity and elegance of this general method which forms a part of the present invention in flow chart form. As shown in Fig. 2, a sample containing a target nucleic acid polymer which includes a region to be sequenced is combined with a reaction mixture containing two primers, a mixture of dNTP's, a chain terminating nucleotide, i.e., a dideoxynucleotide triphosphate, and a thermostable polymerase in a buffer suitable for hybridization and template-dependant polymerization. The mixture is processed for a number of thermal cycles sufficient to produce detectable amounts of sequencing fragments, generally from 20 to 50 cycles. The result of this processing is a product mixture containing dideoxy-terminated fragments which can be loaded directly onto an electrophoresis gel for analysis of the sequence.

A key factor in successfully performing the method of the invention is the utilization of Thermo Sequenase™ or a comparable enzyme as the thermostable polymerase in the reaction mixture. Such an enzyme is characterized by a high affinity for incorporating dideoxynucleotides into the extending nucleotide chain. Thus, for example, Thermo Sequenase is known to favor the incorporation of dideoxynucleotides. In general, for purposes of the present invention, the polymerase used should be one which incorporates dideoxynucleotides into an extending nucleic acid polymer at a rate which is no less than about 0.4 times the rate of incorporation of deoxynucleotides.

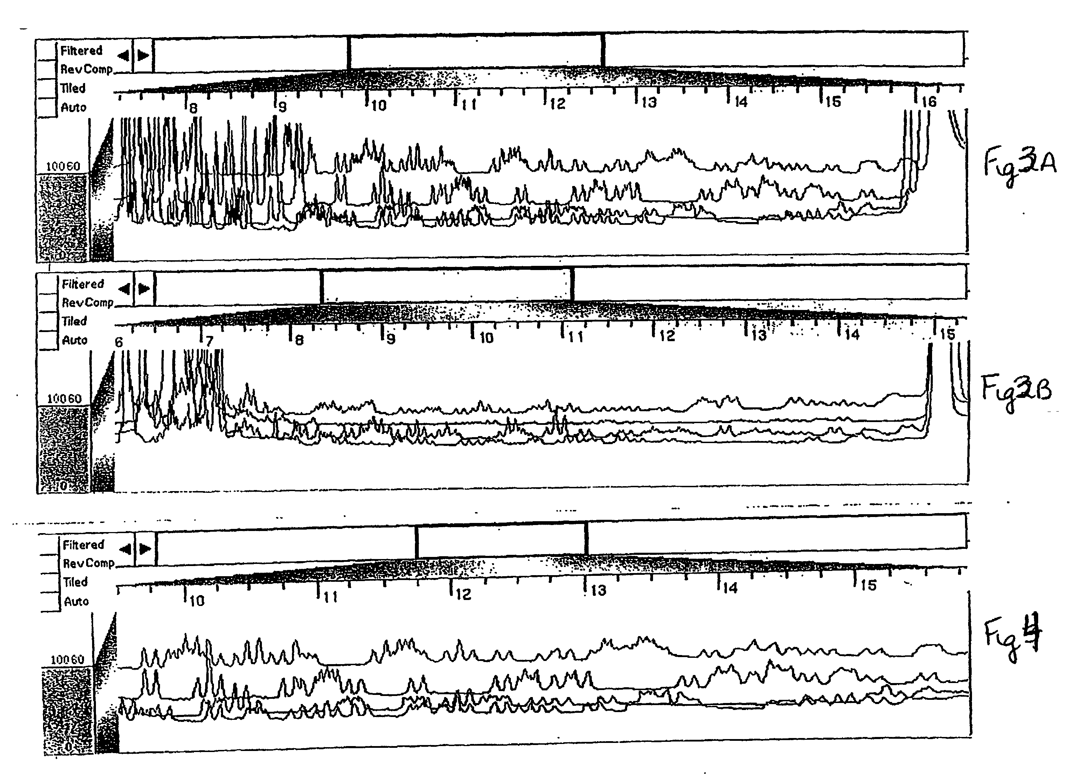

Figs. 3A and 3B and Fig. 4 illustrate the importance of this characteristic of the polymerase enzyme employed. Figs. 3A and 4 shows a sequencing data trace for an actual heterozygous patient sample of natural abundance DNA which was obtained using Thermo Sequenase™ and primers flanking exon 2 of the Von Hippel-Lindau gene in a process according to the invention. Large, well-defined peaks corresponding to the termination fragments were obtained which made sequence evaluation of the sample very straightforward. In addition, the peaks for homozygous peaks are all approximately the same size, and are readily distinguishable from peaks for bases at heterozygous locations. This result was obtained performing the test in a single reaction vessel, with a single unaugmented reaction mixture, in a total of 45 thermal cycles. Comparable results could be obtained using fewer reaction cycles, for example 35 cycles as shown in Example 1 herein.

In contrast, Fig. 3B shows the trace obtained when a combination of Vent and Sequitherm™ were used instead of Thermo Sequenase™ for a total of 45 thermal cycles. In this trace, the peaks for the termination fragments are much smaller and less well defined. Furthermore, the peaks are quite variable in height and did not permit identification of heterozygous peaks based on peak height. Performing the same experiment using Taq polymerase and the high levels of ddNTP's necessary to drive a chain-termination reaction using Taq polymerase resulted in a data trace that contained no usable peaks.

The operation of the sequencing fragment-forming step of the invention can be understood in the context of a hypothetical 200 nt DNA fragment having equal amounts of each base. This means that there will be 50 potential truncation events during the cycle. For each cycle, some of the reaction products would be full length (and thus subject to further reaction with the other primer) and some would be truncated at the points where the ddNTP was added. If each of these truncation events has a statistical likelihood of occurring 1 time in 500 as a result of the relative concentration of ddNTP compared to dNTP and the relative incorporation by the enzyme, then overall a truncation product will occur in slightly less than ten percent of the reactions. Table 1 shows the relative amounts of full-length and chain-termination products theoretically formed after 10, 20 and 30 cycles of reaction using a 200 nt polynucleotide assuming various ratios of truncated to full-length product.

TABLE 1

| Cycles | truncation ratio =0.1 | | truncation ratio =0.3 | | truncation ration = 0.5 | |
|---|---|---|---|---|---|---|
| | truncated | full-length | truncated | full-length | truncated | full-length |
| 10 | 32 | 613 | 86 | 202 | 57 | 57 |
| 20 | 41,000 | 376,000 | 17,400 | 40,462 | 3,300 | 3,300 |
| 30 | $25.6 \times 10^6$ | $230 \times 10^6$ | $3.5 \times 10^6$ | $8.2 \times 10^6$ | 190,000 | 190,000 |

The absolute and relative amounts of nucleotide triphosphates and chain-terminating nucleotide triphosphates may be optimized for the particular enzyme employed. In actual practice, it has been found that useful results are obtained for genomic DNA preparations with Thermo Sequenase™ when the reaction is run for 35 to 45 cycles, using a dideoxy:deoxy mole ratio of 1:100 to 1:300. Fewer cycles may be needed when working with multiplex amplification mixtures, depending on the amount of mixture available for testing.

In general, the nucleotide triphosphates will be included in the reaction mixture at concentrations of from 250 µM to 1.5 mM, and the chain-terminating nucleotide triphosphate will be included at a level of from 0.5 µM to 30 µM to produce compositions in which the mole ratio of the chain terminating nucleotide triphosphate to the corresponding nucleotide triphosphate is from 1:50 to 1:1000, preferably from 1:100 to 1:500. This will result in incorporation of a chain-terminating nucleotide triphosphate into from 30 to 100 percent of the extending polymer chains formed during the thermal cycling of the reaction mixture.

Two groups of oligonucleotide primers are used in the method of the invention. The first group of primers are those employed for multiplex amplification of the selected regions of nucleic acid polymers in the samples. These primers may be selected to amplify several exons of the a gene, regions of several genes from a single organism, or regions of genes from a plurality of microorganisms. While considerable variation is possible in the sequence of the primers used in the multiplex amplification there are certain criteria which should be met.

First each primer pair, i.e., the combination of the 5'-primer and the 3'-primer for any given region, must be unique to that region that only that region gene will be amplified by the primer pair. This means that the primer sequences will be generally somewhat longer than the minimum which can be used as an amplification primer. Preferred primers are from 18 to 23 nucleotides in length, without internal homology or primer-primer homology. It is also desirable for the primers to form more stable duplexes with the target DNA at the primer's 5'-ends than at their 3'-ends, because this leads to less false priming. Stability can be approximated by GC content, since GC base pairs are more stable than AT pairs, or by nearest neighbor thermodynamic parameters. Breslauer et al., "Predicting DNA duplex stability from base sequence", *Proc. Nat'l Acad. Sci. USA* 83: 3746-3750(1986). In addition, to ensure complete amplification of each exon, the two primers of a pair are preferably selected to hybridize in the introns immediately flanking the exon to be amplified using the primer pair.

Additional factors apply to the selection of primers for multiplexed amplification. These factors are discussed in Rylchik, W., Selection of Primers for Polymerase Chain Reaction", in *Methods in Molecular Biology*, *Vol. 15: PCR Protocols: Current Methods and Applications*, White, B.A. ed., Humana Press, Totowa, N.J., 1993. Briefly, applying these factors, primer pairs are selected by position, similarity of melting temperature, internal stability, absence of internal homology or homology to each other, i.e., they won't stick to each other or to themselves, and the 3'-end will not form a stable hairpin loop back on itself. Thus, the goal is to have sets of primer pairs with approximately the same thermal profile, so that they can be effectively coamp-lified together. This goal can be achieved by having groups of primer pairs with approximately the same length and the same G/C content. Finally, it is preferred that the lengths of the amplification product produced by each primer pair to be multiplexed as a group be different from one another. Differences of only one base in length are sufficient, provided a high resolution gel capable of resolving one base differences is used in analyzing the amplification products. However, greater differences in length are preferred.

In addition to the selection of suitable primers, best results in the fragment length analysis are obtained if the amplification reaction is carried out for a limited number of amplification cycles. It will be understood, that the more cycles of amplification are carried out, the more of the desired product will be made and thus the easier its detection will be. It should also be recognized, however, that during the initial cycles (generally the first 20-25 cycles), the amount of DNA of the desired sequence doubles in each cycle, while thereafter the yield of desired product drops off. For maximum effectiveness in the method of the present invention, the amplification of the selected regions should be carried out only for a number of cycles during which doubling of DNA is still being achieved. Such amplification is referred to as "quantitative" amplification.

At least one of the primers of each pair employed in the multiplex amplification step is preferably labeled with a detectable label which permits analysis of the multiplex fragments. The detectable label can be a radiolabel, a fluorophore, a chromophore, a fluorogenic or chromogenic label, or any other label which can facilitate the detection of the amplification products produced in the multiplex reaction.

The other primer of each pair used for multiplex amplification may be unlabeled, or labeled with a detectable label as discussed above. The second primer of each pair may also include a label such as biotin that renders it readily removable from the solution by immobilization on a solid support. As shown in Fig. 1B, a biotin label can be used to capture one strand of each amplified product duplex from the multiplex amplification product, and this mixture is then used as the initial template for the sequencing reaction. This has the advantage of eliminating fluorescently-labeled products from the mixture, permitting the use of the same label for both the sequencing primer and the amplification primers.

The second group of primers used in the invention are a pair of sequencing primers. These primers may be the same as the amplification primers. Preferably, however, the sequencing primers are "nested" primers which produce fragments having a maximum length which is shorter than the multiplex amplification product being sequenced. Utilization of nested primers can also reduce interference caused by labels on the amplification primers where capturable primer labels are not employed.

Like the amplification primers, one or both of the sequencing primers are labeled with a detectable label. The detectable label can be a radiolabel, a fluorophore, a chromophore, a fluorogenic or chromogenic label, or any other label which can facilitate the detection of the sequencing fragments produced. Where both sequencing primers are labeled, the second primer is preferably labeled with a second detectable label that is spectroscopically-distinguishable from the first label. For example, the primers can be labeled with two different fluorophores as in the process described by Wiemann et al., "Simultaneous On-Line DNA Sequencing on Both Stands with Two Fluorescent Dyes," *Anal. Biochem* 224: 117-121 (1995). Analysis of the fragments labeled with the two different labels can be accomplished by loading aliquots of the reaction mixture onto two different electrophoresis lanes which are evaluated for different label types or by loading the product mixture onto one lane in a multi-dye sequencer which has the ability to evaluate several labels in a single instrument.

It may also be advantageous when possible to select a primer sequence which does not include the base corresponding to the ddNTP of the sequencing reaction to avoid truncations within the primer portion of the product polynucleotide. This means that four separate sets of amplification/sequencing primers may need to be constructed for any one region. This is less burdensome than it might seem, however, since in many cases sequencing of only a single base is sufficient for diagnostic purposes. (See US Patent No. 5,834,189).

The basic method of the invention can be enhanced by various modifications without departing from the scope of the present invention. For example, improvements in reproducibility and sensitivity can be obtained by using a combination of an enzyme having a high affinity for incorporation of dideoxynucleotide triphosphates into the extending polymer, e.g., Thermo Sequenase™, and one having a low affinity for incorporation of dideoxynucleotide triphosphates into the extending polymer, e.g., Taq polymerase, under conditions where both enzymes are actively catalyzing template-dependent primer extension polymerization. As noted above, the high affinity enzyme produces almost entirely termination products, with very few of the polymers actually being extended to full length. On the other hand, the low affinity enzyme produces almost exclusively full length product, with relatively few termination products. Addition of the low affinity enzyme to the reaction mixture increases the sensitivity of the method by producing more full length material to be sequenced without increasing the processing time or adding processing steps. The increase in sensitivity can be controlled by varying the ratio of high affinity to low affinity enzyme present in the mixture.

It will be noted, however, that including of low affinity enzyme to produce full length product will also result in the formation of a very intense labeled full-length product peak. This peak may make analysis of the bases near the end of the sequence difficult. To obtain the benefits of increased sensitivity while making less full length product, it may be desirable to utilize a low affinity enzyme which is more thermolabile than Taq polymerase, such that the low affinity enzyme is essentially inactivated by the end of the first 15 to 25 cycles. This would allow the production of longer fragments early in the assay and the generation of more terminated fragments late in the assay.

The reaction mixture of the invention may also incorporate other additives which enhance the formation of sequencing fragments. For example, a product called TaqStart™ Antibody is a monoclonal antibody which binds to and blocks the activities of Taq polymerase. This antibody is added to PCR reactions using Taq polymerase to block enzyme activity during set-up at ambient temperature to prevent or reduce the formation of non-specific amplification products. TaqStart™ Antibody can be used in the present invention with Thermo Sequenase™ to reduce nonspecific primer extension reactions.

Other materials which can be used in the reaction mixture of the invention are uracil-DNA glycosylases and corresponding unconventional nucleotides as described in US Patent No. 5,418,149, to reduce non-specific product formation. Roche sells a product under the trademark AMPERASE™ which can be used ocnveniently for this purpose.

EXAMPLE 1

A 175 ng sample of genomic DNA prepared from a patient blood sample using the Gentra™ Pure Gene DNA isolation kit. Briefly, in this procedure the blood cells were lysed, centrifuged to recover the lysed white blood cells and mixed with proteinase K. Protein is then separated from the sample by precipitation and the remaining nucleic acids are precipitated and collected. The resulting genomic DNA preparation was combined with two primers effective to amplify exon 2 of the VHL gene.

5' primer - labeled with the fluorophore Cy5;

**GGCTCTTTAA CAACCTTT** [SEQ ID No.: 1]

3' primer - unlabeled;

**GGGCTTAATT TTTCAAGTGG TC** [SEQ ID No.: 2]

The reaction mixture employed had the following composition:

|  | final amt. | final vol. |
|---|---|---|
| DNA genomic | 175 ng | 3.5 ul |
| 5' Primer        1pM/ul | 3 pMol | 3.0 ul |
| 3' Primer)        7.5pM/ul | 23 pMol | 3.0 ul |
| DMSO        100% |  | 1.5 ul |
| ThermoSequenase Reaction Buffer |  | 2.0 ul |
| ThermoSequenase Enzyme 32U/ul | 6.4 U | 0.2 ul |
|  | Total | 13.2 ul |

3 ul aliquots of the reaction mixture were placed into each of 4 tubes containing 3 ul of one of the following termination mixes: A, C, G or T (dNTP/ddNTP; 100:1 ratio; 750 microM: 7.5 microM)after which the mixture was layered with oil.

The mixture was then processed in PTC 100 Thermocycler at follows:

| denature | |
|---|---|
| 95° | 120 sec |
| **35 cycles** | |
| 95° | 50 sec |
| 52° | 30 sec |
| 70° | 60 sec |
| **finish** | |
| 70° | 120 sec |

(continued)

| finish | |
|---|---|
| 6° | soak |

6 ul dye/stop solution was then added to each tube to a final volume of 12 ul. A 2 ul sample was then loaded onto a thin polyacrylamide gel and analyzed in a MicroGene Blaster sequencer (Visible Genetics Inc, Toronto, Canada). The result was a clean, easily interpreted sequencing ladder.

EXAMPLE 2

A 500 ng sample of genomic DNA prepared from a patient sample using a standard SDS-Proteinase K-phenol extraction was combined with the same two primers as in Example 1 flanking exon 2 of the VHL gene. The reaction mixture employed had the following composition:

| | final amt. | final vol. |
|---|---|---|
| DNA genomic | 500 ng | 1.0 ul |
| 5' Primer        3 pMol | | 3.0 ul |
| 3' Primer        3 pMol | | 3.0 ul |
| DMSO        100% | | 1.5 ul |
| ThermoSequenase Reaction Buffer | | 2.0 ul |
| ThermoSequenase Enzyme 32U/ul | 6.4 U | 0.2 ul |
| distilled water | | 3.0 ul |
| | Total Volume | 13.2 ul |

3 ul aliquots of the reaction mixture were placed into each of 4 tubes containing 3 ul of one of the following termination mixes: A, C, G or T (dNTP/ddNTP; 100:1 ratio; 750 microM: 7.5 microM) after which the mixture was layered with oil.
The mixture was then processed in PTC 100 Thermocycler at follows:

| denature | |
|---|---|
| 94° | 120 sec |
| 45 cycles | |
| 94° | 20 sec |
| 52° | 20 sec |
| 72° | 20 sec |
| finish | |
| 72° | 150 sec |
| 6° | soak |

6 ul dye/stop solution was then added to each tube to a final volume of 12 ul. A 1 ul sample was then loaded onto a thin polyacrylamide gel and analyzed in a MicroGene Blaster sequencer (Visible Genetics Inc, Toronto, Canada).
For comparison, a sample of the same genomic DNA was treated in a similar reaction using a mixture of Vent Enzyme and Sequitherm Enzyme as follows:

| | final amt. | final vol. |
|---|---|---|
| DNA genomic | 1 ug | 8.0 ul |
| 5' Primer (Cy5.5 labeled) | 12.5 pMol | 12.5 ul |
| 3' Primer (unlabeled) | 12.5 pMol | 12.5 ul |
| Triton X-100        20% | | 25 ul |
| 1 mM each dNTP | | 4.0 ul |
| 10X Vent Buffer | | 5.0 ul |

(continued)

|  | final amt. | final vol. |
|---|---|---|
| 10X Sequitherm Buffer |  | 5.0 ul |
| Vent Enzyme 2U/ul |  | 2.0 ul |
| Sequitherm Enzyme 5 U/ul |  | 2.0 ul |
| distilled water |  | 6.5 ul |
|  | Total Volume | 82.5 ul |

20 ul of reaction mixture of aliquoted into each of 4 tubes containing 5 ul of one of the following ddNTP in water:

| ddATP | 850 uM |
|---|---|
| ddCTP | 500 uM |
| ddGTP | 100 uM |
| ddTTP | 1700 uM |

and layered with oil. These mixtures were processed in a PTC 100 thermocycler as follows:

| denature | |
|---|---|
| 94° | 90 sec |
| 45 cycles | |
| 94° | 20 sec |
| 52° | 20 sec |
| 72° | 20 sec |
| finish | |
| 72° | 150 sec |
| 6° | soak |

25 ul of dye/stop solution as then added to each tube to a final volume of 50 ul. 2 ul aliquots of this solution were loaded onto a MicroGene Blaster sequencer for analysis.

The sequencing traces taken for these experiments are shown in Figs. 3A-B and 4. Figs. 3A and Fig. 4 show the result for the Thermo Sequenase™ runs according to the invention is vastly superior to the comparative tests even though a smaller volume of initial DNA was used. In particular, not only are the peaks more detectable, the peaks in the ThermoSequenase™ run are also correctly reflect the fact that the sample was a heterozygote, providing peaks of substantially uniform size to reflect one versus two bases. This makes analysis of the results much easier. Thus, these experiments demonstrate the surprising characteristics of the method of the invention.

EXAMPLE 3

For comparison to the method of the invention, an experiment was conducted in which the ability of Taq Polymerase to produce usable sequencing fragments directly from genomic DNA was tested. No sequence information could be obtained using Taq polymerase.

EXAMPLE 4

A patient sample is amplified in a multiplex amplification process using the methods and primers disclosed in International Patent Publication No. WO 96/01909. Thereafter, the sequence of one or more exons of the p53 gene is determined by incorporating an aliquot of the multiplex amplification reaction product in a Pre-Reaction Mixture containing:

| Multiplex Amplification Product (25 to 100 ng DNA/ul) | 3.0 ul |
|---|---|
| 1:10 Diluted ThermoSequenase Enzyme (Amersham) | 2.0 ul |
| 10X Enzyme Reaction Buffer (Amersham) | 2.0 ul |

(continued)

| Primer A | (10 uM) | 0.6 ul |
|---|---|---|
| Primer B | (10 uM) | 0.6 ul |
| DMSO | | 1.3 ul |
| ddH2O | | 3.5 ul |
| | | 13.0 ul |

The primers employed as Primer A and Primer B depend on the specific p53 exon sequence desired. A non-exclusive list of primers useful in practicing the invention are set out in Table 2. At least one of the primers must be labeled with a detectable label if used in a fluorescence-based automated DNA sequencer.

Separate termination mixtures are also prepared using a ratio of deoxynucleoside triphosphates (dNTPs) to dideoxynucleotide triphosphates (ddNTPs) of 300 : 1.

A Termination Mix: 750 uM each dNTP (dATP, dCTP, dGTP, dTTP); 2.5 uM ddATP
C Termination Mix: 750 uM each dNTP (dATP, dCTP, dGTP, dTTP); 2.5 uM ddCTP
G Termination Mix: 750 uM each dNTP (dATP, dCTP, dGTP, dTTP); 2.5 uM ddGTP
T Termination Mix: 750 uM each dNTP (dATP, dCTP, dGTP, dTTP); 2.5 uM ddTTP
3 ul of the Pre-Reaction Mixture is added to 3 ul of each Termination Mix, and mixed well. This Reaction Mixture is then treated to the following temperature cycles in an automated thermocycler (such as the MJ Research PTC-100 Programmable Thermal Controller):

| 94°C | 5 min |
|---|---|
| then 40 cycles of | |
| 94°C | 30 sec |
| 60°C | 30 sec |
| 70°C | 60 sec |

and a final extension reaction of 5 mins at 70°C. The Reaction Mixture is then placed on ice. 6 ul of STOP/Loading buffer (100% formamide; colored dye) is added and mixed. 1.5 ul of the final mixture is loaded in a single well of a MicroGene Blaster automated DNA sequencer (Visible Genetics Inc., Toronto). The reaction products are separated by electrophoresis and detected. GeneObjects software (Visible Genetics Inc.) is used to analyze the results and present the sequence. The results are reported to the patient file.

## TABLE 2

(primer and exon locations are given using nucleotide numbers in the sequence of p53 (GeneBank Accession No. 54156) submitted by Chumakov et al.

| Exon | Sequence | Primer Location | Fragment Size | Exon Location | Primer Conc (umol/L) |
|---|---|---|---|---|---|
| 1 | CGGATTACTT GCCCTTACTT GTCA [SEQ 3] | 711 | 331 | 843-949 | 0.4 |
| | CCCCAGCCCC AGCGATTTT [SEQ 4] | 1041 | | | |
| 2 | CCAGGGTTGG AAGCGTCTC [SEQ 5] | 11641 | 259 | 11689-11790 | 0.9 |
| | GACAAGAGCA GAAAGTCAGTCC [SEQ 6] | 11899 | | | |
| 3 | CATGGGACTG ACTTTCTGCT [SEQ 7] | 11874 | 141 | 11906-11927 | 0.8 |
| | ATGGGTGAAA AGAGCAGT [SEQ 8] | 12014 | | | |
| 4 | CTGGTCCTCT GACTGCTCTT TTCA [SEQ 9] | 11986 | 382 | 12021-12299 | 0.48 |
| | AAAGAAATGC AGGGGGATAC GG [SEQ 10] | 12367 | | | |
| 5 | TGTTCACTTG TGCCCTGACT [SEQ 11] | 13005 | 268 | 13055-13432 | 0.2 |
| | CAGCCCTGTC GTCTCTCCAG [SEQ 12] | 13272 | | | |
| 6 | CTGGGGCTGG AGAGACGACA [SEQ 13] | 13247 | 274 | 13320-13432 | 0.14 |
| | GGAGGGCCAC TGACAACCA [SEQ 14] | 13493 | | | |
| 7 | CTCCCCTGCT TGCCACA [SEQ 15] | 13933 | 245 | 14000-14109 | 0.4 |
| | GGGTCAGCGG CAAGCAGAGG [SEQ 16] | 14177 | | | |
| 8 | GACAAGGGTG GTTGGGAGTA GATG [SEQ 17] | 14350 | 320 | 14452-14588 | 0.2 |
| | GCAAGGAAAG GTGATAAAAG TGAA [SEQ 18] | 14669 | | | |

| TABLE 2 | | | | | | |
|---|---|---|---|---|---|---|
| (primer and exon locations are given using nucleotide numbers in the sequence of p53 (GeneBank Accession No. 54156) submitted by Chumakov et al. | | | | | | |
| Exon | Sequence | Primer Location | Fragment Size | Exon Location | Primer Conc (umol/L) | |
| 9 | GCGGTGGAGG AGACCAAGG [SEQ 19] | 14609 | 209 | 14681-14754 | 0.1 | |
| | AACGGCATTT TGAGTGTTAG AC [SEQ 20] | 14817 | | | | |
| 10 | TGATCCGTCA TAAAGTCAAA CAA [SEQ 21] | 17477 | 390 | 17572-17678 | 0.3 | |
| | GTGGAGGCAA GAATGTGGTT A [SEQ 22] | 17866 | | | | |
| 11 | GGCACAGACC CTCTCACTCA T [SEQ 23] | 18540 | 256 | 18599-18876 | 0.4 | |
| | TGCTTCTGAC GCACACCTAT T [SEQ 24] | 18795 | | | | |

EXAMPLE 5

A patient sample is amplified in a multiplex amplification process using the methods and primers disclosed in International Patent Publication No. WO 96/01908. Thereafter, the sequence of one or more exons of the RB1 gene is determined by incorporating an aliquot of the multiplex amplification reaction product in a Pre-Reaction Mixture as in Example 4, but using a pair of sequencing primers for RB1. Suitable pairs of sequencing primers are the 3' and 5' sequencing primers for each exon disclosed in WO 96/01908.

SEQUENCE LISTING

1) GENERAL INFORMATION:

(I) APPLICANT: Visible Genetics Inc.

Leushner, James
Hui, May
Dunn, James M.
Larson, Marina T.
Jean-Michel Lacroix

(ii) TITLE OF INVENTION: METHOD FOR DETERMINATION OF NUCLEIC CID SEQUENCES AND DIAGNOSTIC APPLICATIONS THEREOF

(iii) NUMBER OF SEQUENCES: 24
(iv) CORRESPONDENCE ADDRESS:

(A) ADDRESSEE: Oppedahl & Larson
(B) STREET: 1992 Commerce Street Suite 309
(C) CITY: Yorktown

(D) STATE: NY
(E) COUNTRY: US
(F) ZIP: 10598

(V) COMPUTER READABLE FORM:

(A) MEDIUM TYPE: Diskette - 3.5 inch, 1.44 Mb storage
(B) COMPUTER: IBM compatible
(C) OPERATING SYSTEM: MS DOS
(D) SOFTWARE: Word Perfect

(vi) CURRENT APPLICATION DATA :

(A) APPLICATION NUMBER:
(B) FILING DATE:
(C) CLASSIFICATION:

(vii) PRIOR APPLICATION DATA:

(A) APPLICATION NUMBER:
(B) FILING DATE:

(viii) ATTORNEY/AGENT INFORMATION :

(A) NAME: Larson, Marina T.
(B) REGISTRATION NUMBER: 32,038
(C) REFERENCE/DOCKET NUMBER: VGEN.P-020-WO

(ix) TELECOMMUNICATION INFORMATION:

(A) TELEPHONE: (914) 245-3252
(B) TELEFAX: (914) 962-4330
(C) TELEX:

2) INFORMATION FOR SEQ ID NO: 1:

(I) SEQUENCE CHARACTERISTICS:

(A) LENGTH: 18
(B) TYPE: nucleic acid
(C) STRANDEDNESS: double
(D) TOPOLOGY: linear

(ii) MOLECULE TYPE: other nucleic acid
(iii) HYPOTHETICAL: NO
(iv) ANTI-SENSE: yes
(v) FRAGMENT TYPE: internal
(vi) ORIGINAL SOURCE:

(A) ORGANISM: human
(D) OTHER INFORMATION: amplification primer for VHL exon 2

(xi) SEQUENCE DESCRIPTION: SEQ ID NO:1:


GGCTCTTTAA CAACCTTT        18

(2) INFORMATION FOR SEQ ID NO: 2:

(I) SEQUENCE CHARACTERISTICS:

    (A) LENGTH: 22
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

    (A) ORGANISM: human
    (D) OTHER INFORMATION: amplification primer for VHL exon 2

(xi) SEQUENCE DESCRIPTION: SEQ ID NO:2:

GGGCTTAATT TTTCAAGTGG TC        22

(2) INFORMATION FOR SEQ ID NO: 3:

(I) SEQUENCE CHARACTERISTICS:

    (A) LENGTH: 24
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

    (A) ORGANISM: human
    (D) OTHER INFORMATION: sequencing primer for p53 exon 1

(xi) SEQUENCE DESCRIPTION: SEQ ID NO:3:

CGGATTACTT GCCCTTACTT GTCA      24

(2) INFORMATION FOR SEQ ID NO: 4:

(I) SEQUENCE CHARACTERISTICS:

    (A) LENGTH: 19
    (B) TYPE: nucleic acid

(C) STRANDEDNESS: double
(D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

(A) ORGANISM: human
(D) OTHER INFORMATION: sequencing primer for p53 exon 1

(xi) SEQUENCE DESCRIPTION: SEQ ID NO:4:

CCCCAGCCCC AGCGATTTT 19

(2) INFORMATION FOR SEQ ID NO: 5:

(I) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

(A) ORGANISM: human
(D) OTHER INFORMATION: sequencing primer for p53 exon 2

(xi) SEQUENCE DESCRIPTION: SEQ ID NO:5:

CCAGGGTTGG AAGCGTCTC 19

(2) INFORMATION FOR SEQ ID NO: 6:

(I) SEQUENCE CHARACTERISTICS:

(A) LENGTH: 22
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: no
(v) FRAGMENT TYPE: internal

(vi) ORIGINAL SOURCE:

    (A) ORGANISM: human
    (D) OTHER INFORMATION: sequencing primer for p53 exon 2

(xi) SEQUENCE DESCRIPTION: SEQ ID NO:6:

GACAAGAGCA GAAAGTCAGT CC       22

(2) INFORMATION FOR SEQ ID NO: 7:

(I) SEQUENCE CHARACTERISTICS:

    (A) LENGTH: 20
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

    (A) ORGANISM: human
    (D) OTHER INFORMATION: sequencing primer for p53 exon 3

(xi) SEQUENCE DESCRIPTION: SEQ ID NO:7:

CATGGGACTG ACTTTCTGCT       20

(2) INFORMATION FOR SEQ ID NO: 8:

(I) SEQUENCE CHARACTERISTICS:

    (A) LENGTH: 18
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

    (A) ORGANISM: human
    (D) OTHER INFORMATION: sequencing primer for p53 exon 3

(xi) SEQUENCE DESCRIPTION: SEQ ID NO:8:

ATGGGTGAAA AGAGCAGT        18

(2) INFORMATION FOR SEQ ID NO: 9:

    (I) SEQUENCE CHARACTERISTICS:

        (A) LENGTH: 24
        (B) TYPE: nucleic acid
        (C) STRANDEDNESS: double
        (D) TOPOLOGY: linear

    (ii) MOLECULE TYPE: other nucleic acid
    (iii) HYPOTHETICAL: No
    (iv) ANTI-SENSE: yes
    (v) FRAGMENT TYPE: internal
    (vi) ORIGINAL SOURCE:

        (A) ORGANISM: human
        (D) OTHER INFORMATION: sequencing primer for p53 exon 4

    (xi) SEQUENCE DESCRIPTION: SEQ ID NO:9:

CTGGTCCTCT GACTGCTCTT TTCA        24

(2) INFORMATION FOR SEQ ID NO: 10:

    (I) SEQUENCE CHARACTERISTICS:

        (A) LENGTH: 22
        (B) TYPE: nucleic acid
        (C) STRANDEDNESS: double
        (D) TOPOLOGY: linear

    (ii) MOLECULE TYPE: other nucleic acid
    (iii) HYPOTHETICAL: No
    (iv) ANTI-SENSE: no
    (v) FRAGMENT TYPE: internal
    (vi) ORIGINAL SOURCE:

        (A) ORGANISM: human
        (D) OTHER INFORMATION: sequencing primer for p53 exon 4
        (xi) SEQUENCE DESCRIPTION: SEQ ID NO:10:

AAAGAAATGC AGGGGGATAC GG        22

(2) INFORMATION FOR SEQ ID NO: 11:

    (I) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

(A) ORGANISM: human
(D) OTHER INFORMATION: sequencing primer for p53 exon 5

(xi) SEQUENCE DESCRIPTION: SEQ ID NO:11:

TGTTCACTTG TGCCCTGACT          20

(2) INFORMATION FOR SEQ ID NO: 12:

(I) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

(A) ORGANISM: human
(D) OTHER INFORMATION: sequencing primer for p53 exon 5

(xi) SEQUENCE DESCRIPTION: SEQ ID NO:12:

CAGCCCTGTC GTCTCTCCAG          20

(2) INFORMATION FOR SEQ ID NO: 13:

(I) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: yes

(v) FRAGMENT TYPE: internal
(vi) ORIGINAL SOURCE:

    (A) ORGANISM: human
    (D) OTHER INFORMATION: sequencing primer for p53 exon 6

(xi) SEQUENCE DESCRIPTION: SEQ ID NO:13:

```
CTGGGGCTGG AGAGACGACA           20
```

(2) INFORMATION FOR SEQ ID NO: 14:

(I) SEQUENCE CHARACTERISTICS:

    (A) LENGTH: 19
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

    (A) ORGANISM: human
    (D) OTHER INFORMATION: sequencing primer for p53 exon 6

(xi) SEQUENCE DESCRIPTION: SEQ ID NO:14:

```
GGAGGGCCAC TGACAACCA          19
```

(2) INFORMATION FOR SEQ ID NO: 15:

(I) SEQUENCE CHARACTERISTICS:

    (A) LENGTH: 17
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

    (A) ORGANISM: human
    (D) OTHER INFORMATION: sequencing primer for p53 exon 7

(xi) SEQUENCE DESCRIPTION: SEQ ID NO:15:

CTCCCCTGCT TGCCACA          17

(2) INFORMATION FOR SEQ ID NO: 16:

(I) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

(A) ORGANISM: human
(D) OTHER INFORMATION: sequencing primer for p53 exon 7

(xi) SEQUENCE DESCRIPTION: SEQ ID NO:16:

GGGTCAGCGG CAAGCAGAGG          20

(2) INFORMATION FOR SEQ ID NO: 17:

(I) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

(A) ORGANISM: human
(D) OTHER INFORMATION: sequencing primer for p53 exon 8

(xi) SEQUENCE DESCRIPTION: SEQ ID NO:17:

GACAAGGGTG GTTGGGAGTA GATG          24

(2) INFORMATION FOR SEQ ID NO: 18:

(I) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

(A) ORGANISM: human
(D) OTHER INFORMATION: sequencing primer for p53 exon 8

(xi) SEQUENCE DESCRIPTION: SEQ ID NO:18:

```
GCAAGGAAAG GTGATAAAAG TGAA          24
```

(2) INFORMATION FOR SEQ ID NO: 19:

(I) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

(A) ORGANISM: human
(D) OTHER INFORMATION: sequencing primer for p53 exon 9

(xi) SEQUENCE DESCRIPTION: SEQ ID NO:19:

```
GCGGTGGAGG AGACCAAGG          19
```

(2) INFORMATION FOR SEQ ID NO: 20:

(I) SEQUENCE CHARACTERISTICS:

(A) LENGTH: 22
(B) TYPE: nucleic acid
(C) STRANDEDNESS: double
(D) TOPOLOGY: linear

(ii) MOLECULE TYPE: other nucleic acid
(iii) HYPOTHETICAL: No
(iv) ANTI-SENSE: no

(v) FRAGMENT TYPE: internal
(vi) ORIGINAL SOURCE:

    (A) ORGANISM: human
    (D) OTHER INFORMATION: sequencing primer for p53 exon 9

(xi) SEQUENCE DESCRIPTION: SEQ ID NO:20:

**AACGGCATTT TGAGTGTTAG AC**        **22**

(2) INFORMATION FOR SEQ ID NO: 21:

(I) SEQUENCE CHARACTERISTICS:

    (A) LENGTH: 23
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

    (A) ORGANISM: human
    (D) OTHER INFORMATION: sequencing primer for p53 exon 10

(xi) SEQUENCE DESCRIPTION: SEQ ID NO:21:

**TGATCCGTCA TAAAGTCAAA CAA**        **23**

(2) INFORMATION FOR SEQ ID NO: 22:

(I) SEQUENCE CHARACTERISTICS:

    (A) LENGTH: 21
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

    (A) ORGANISM: human
    (D) OTHER INFORMATION: sequencing primer for p53 exon 10

(xi) SEQUENCE DESCRIPTION: SEQ ID NO:22:

```
GTGGAGGCAA GAATGTGGTT A          21
```

(2) INFORMATION FOR SEQ ID NO: 23:

(I) SEQUENCE CHARACTERISTICS:

    (A) LENGTH: 21
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: yes
(v) FRAGMENT TYPE: internal
(vi) ORIGINAL SOURCE:

    (A) ORGANISM: human
    (D) OTHER INFORMATION: sequencing primer for p53 exon 11

(xi) SEQUENCE DESCRIPTION: SEQ ID NO:23:

```
GGCACAGACC CTCTCACTCA T          21
```

(2) INFORMATION FOR SEQ ID NO: 24:

(I) SEQUENCE CHARACTERISTICS:

    (A) LENGTH: 21
    (B) TYPE: nucleic acid
    (C) STRANDEDNESS: double
    (D) TOPOLOGY: linear

(ii) MOLECULE TYPE: other nucleic acid
(iii) HYPOTHETICAL: No
(iv) ANTI-SENSE: no
(v) FRAGMENT TYPE: internal
(vi) ORIGINAL SOURCE:

    (A) ORGANISM: human
    (D) OTHER INFORMATION: sequencing primer for p53 exon 11

(xi) SEQUENCE DESCRIPTION: SEQ ID NO:24:

```
TGCTTCTGAC GCACACCTAT T          21
```

**Claims**

1. A method for analyzing a nucleic acid-containing sample comprising the steps of performing a multiplex amplification reaction on the nucleic acid-containing sample using a plurality of amplification primer pairs, one pair for each of a plurality of regions to be analyzed, to produce a mixture of amplified fragments and determining the sequence of at least one species of amplified fragment, **characterized in that** the sequence is determined by combining the mixture of amplification fragments with a sequencing reaction mixture for the production of sequencing fragments and evaluating the sequencing fragments produced therefrom.

2. The method of claim 1, **characterized in that** at least one aliquot of the mixture of amplified fragments is combined with a sequencing mixture comprising

first and second sequencing primers,

a nucleotide triphosphate feedstock mixture,

a chain-terminating nucleotide triphosphate, and

a thermally stable polymerase enzyme which incorporates dideoxynucleotides into an extending nucleic acid polymer at a rate which is no less than 0.4 times the rate of incorporation of deoxynucleotides to form a sequencing reaction mixture, said first and second sequencing primers binding to the sense and antisense strands, respectively, of the amplified fragments from a selected one of the regions; that the sequencing reaction mixture is exposed to a plurality of temperature cycles each of which includes at least a high temperature denaturation phase and a lower temperature extension phase, thereby producing a plurality of terminated fragments; and that the terminated fragments are evaluated to determine the position of the base corresponding to the chain-terminating nucleotide triphosphate within the selected fragment.

3. The method according to claim 2, wherein at least one primer of each pair of amplification primers used in the multiplex amplification reaction is labeled with a capturable label, and wherein the amplified fragments are captured on a solid support and washed prior to combining them with the sequencing mixture.

4. The method according to claim 3, wherein the capturable label is biotin.

5. The method according to any of claims 2 to 4, wherein the thermostable polymerase enzyme is Thermo Sequenase™.

6. The method according to any of claim 2 to 5, wherein at least one of the first and second sequencing primers in the sequencing mixture is labeled with a fluorescent label.

7. The method according to any of claims 2 to 6, wherein the first and second sequencing primers in the sequencing mixture are each labeled with a different spectroscopically-distinguishable fluorescent label.

8. The method according to any of claims 2 to 7, wherein the sequencing mixture further comprises a second polymerase enzyme having a low affinity for incorporation of dideoxynucleotide triphosphates compared to deoxynucleotide triphosphates.

9. The method according to claim 8, wherein the second polymerase is Taq polymerase.

10. The method according to any of claims 1 to 7, wherein the species of amplified fragments produced by the pairs of amplification primers each have a different length.

## Patentansprüche

1. Verfahren zum Analysieren einer Nukleinsäure-haltigen Probe, umfassend die Schritte: Durchführen einer Mehrfach-Amplifikationsreaktion bei der Nukleinsäure-haltigen Probe unter Verwendung einer Mehrzahl von Amplifikations-Primerpaaren, ein Paar für jede einer Mehrzahl von zu analysierenden Regionen, um eine Mischung von amplifizierten Fragmenten zu erzeugen, und Bestimmen der Sequenz mindestens einer Spezies von amplifiziertem Fragment, **dadurch gekennzeichnet, dass** die Sequenz durch Vereinigen der Mischung von Amplifikationsfragmenten mit einer Sequenzierungs-Reaktionsmischung für die Erzeugung von Sequenzierungsfragmenten und Auswerten der daraus erzeugten Sequenzierungsfragmente bestimmt wird.

2. Verfahren nach Anspruch 1, **dadurch gekennzeichnet, dass** mindestens eine Aliquote der Mischung von amplifizierten Fragmenten mit einer Sequenzierungsmischung vereinigt wird, welche umfasst:

einen ersten und einen zweiten Sequenzierungsprimer,

eine Nukleotidtriphosphat-Eduktmischung,

ein Kettenabbruchs-Nukleotidtriphosphat und

ein thermisch stabiles Polymeraseenzym, welches Didesoxynukleotide in ein sich verlängerndes Nukleinsäure-Polymer bei einer Geschwindigkeit einbaut, die nicht weniger als das 0,4-fache der Geschwindigkeit des Einbaus von Desoxynukleotiden ist, um eine Sequenzierungs-Reaktionsmischung zu bilden, wobei sich der erste und der zweite Sequenzierungsprimer an die Sinn- bzw. Antisinnstränge der amplifizierten Fragmente aus einer ausgewählten der Regionen binden; dass die Sequenzierungs-Reaktionsmischung einer Mehrzahl von Temperaturzyklen ausgesetzt wird, von denen jeder mindestens eine Hochtemperatur-Denaturierungsphase und eine Verlängerungsphase bei niedriger Temperatur einschließt, wodurch eine Mehrzahl von abgebrochenen Fragmenten erzeugt wird; und dass die abgebrochenen Fragmente ausgewertet werden, um die Position der Base, die dem Kettenabbruchs-Nukleotidtriphosphat entspricht, innerhalb des ausgewählten Fragments zu bestimmen.

3. Verfahren nach Anspruch 2, in dem mindestens ein Primer von jedem Paar Amplifikationsprimern, die in der Mehrfach-Amplifikationsreaktion verwendet werden, mit einem einfangbaren Marker markiert ist und in dem die amplifizierten Fragmente auf einem festen Träger eingefangen und gewaschen werden, bevor sie mit der Sequenzierungsmischung vereinigt werden.

4. Verfahren nach Anspruch 3, in dem der einfangbare Marker Biotin ist.

5. Verfahren nach irgendeinem der Ansprüche 2 bis 4, in dem das thermostabile Polymeraseenzym Thermo Sequenase™ ist.

6. Verfahren nach irgendeinem der Ansprüche 2 bis 5, in dem mindestens einer aus dem ersten und zweiten Sequenzierungsprimer in der Sequenzierungsmischung mit einem fluoreszierenden Marker markiert ist.

7. Verfahren nach irgendeinem der Ansprüche 2 bis 6, in dem der erste und der zweite Sequenzierungsprimer in der Sequenzierungsmischung jeweils mit einem unterschiedlichen, spektroskopisch unterscheidbaren fluoreszierenden Marker markiert ist.

8. Verfahren nach irgendeinem der Ansprüche 2 bis 7, in dem die Sequenzierungsmischung weiter ein zweites Polymeraseenzym mit einer niedrigen Affinität zum Einbau von Didesoxynukleotidtriphosphaten, verglichen mit Desoxynukleotidtriphosphaten, umfasst.

9. Verfahren nach Anspruch 8, in dem die zweite Polymerase Taq-Polymerase ist.

10. Verfahren nach irgendeinem der Ansprüche 1 bis 7, in dem die Spezies von amplifizierten Fragmenten, die durch die Paare von Amplifikationsprimern erzeugt werden, jeweils eine andere Länge aufweisen.

## Revendications

1. Procédé pour analyser un échantillon contenant des acides nucléiques comprenant les étapes de réalisation d'une réaction d'amplification multiplex sur l'échantillon contenant des acides nucléiques au moyen d'une pluralité de paires d'amorces d'amplification, une paire pour chaque région d'une pluralité de régions à analyser, pour produire un mélange de fragments amplifiés et de détermination de la séquence d'au moins une espèce de fragment amplifié, **caractérisé en ce que** la séquence est déterminée par combinaison du mélange de fragments d'amplification avec un mélange réactionnel de séquençage pour la production de fragments de séquençage et évaluation des fragments de séquençage ainsi produits.

2. Procédé selon la revendication 1, **caractérisé en ce qu'**au mois une portion aliquote du mélange de fragments amplifiés est combinée avec un mélange de séquençage comprenant

des première et seconde amorces de séquençage,

un mélange de charge de nucléotides triphosphates,

un nucléotide triphosphate de terminaison de chaîne, et

une enzyme polymérase thermiquement stable qui incorpore des didésoxynucléotides dans un polymère d'acide nucléique en extension à un taux qui est au moins égal à 0,4 fois le taux d'incorporation de désoxynucléotides pour former un mélange réactionnel de séquençage, lesdites première et seconde amorces de séquençage se liant aux brins sens et anti-sens, respectivement, des fragments amplifiés provenant d'une région choisie parmi les régions ; **en ce que** le mélange réactionnel de séquençage est exposé à une pluralité de cycles de température incluant chacun au moins une phase de dénaturation à haute température et une phase d'extension à plus basse température, pour produire une pluralité de fragments terminés ; et **en ce que** les fragments terminés sont évalués pour déterminer la position de la base correspondant au nucléotide triphosphate de terminaison de chaîne dans le fragment choisi.

3. Procédé selon la revendication 2, où au moins une amorce de chaque paire d'amorces d'amplification utilisées dans la réaction d'amplification multiplex est marquée avec un marqueur capturable, et où les fragments amplifiés sont capturés sur un support solide et lavés avant d'être combinés avec le mélange de séquençage.

4. Procédé selon la revendication 3, où le marqueur capturable est la biotine.

5. Procédé selon l'une quelconque des revendications 2 à 4, où l'enzyme polymérase thermostable est la Thermo Sequenase™.

6. Procédé selon l'une quelconque des revendications 2 à 5, où au moins l'une des première et seconde amorces de séquençage dans le mélange de séquençage est marquée avec un marqueur fluorescent.

7. Procédé selon l'une quelconque des revendications 2 à 6, où les première et seconde amorces de séquençage dans le mélange de séquençage sont marquées chacune avec un marqueur fluorescent pouvant être distingué par spectroscopie différent.

8. Procédé selon l'une quelconque des revendications 2 à 7, où le mélange de séquençage comprend en outre une seconde enzyme polymérase ayant une faible affinité pour l'incorporation de didésoxynucléotides triphosphates par rapport aux désoxynucléotides triphosphates.

9. Procédé selon la revendication 8, où la seconde polymérase est la Taq polymérase.

10. Procédé selon l'une quelconque des revendications 1 à 7, où les espèces de fragments amplifiés produits par les paires d'amorces d'amplification ont chacune une longueur différente.

**FIG. 1A**

FIG. 1B

EP 0 907 752 B1

REACTION MIXTURE+CHAIN TERMINATING NUCLEOTIDE+
NATURAL ABUNDANCE SAMPLE

THERMAL CYCLES TO FORM
DETECTABLE AMOUNT OF
TERMINATED FRAGMENTS FOR
ANALYSIS

PRODUCT FOR LOADING ON GEL

FIG. 2

Fig 3A

Fig 3B

Fig 4

EP 0 907 752 B1